(19) Europäisches Patentamt
European Patent Office
Office européen des brevets

(11) Publication number : **0 672 652 A1**

(12) **EUROPEAN PATENT APPLICATION**

(21) Application number : **95810154.5**

(22) Date of filing : **09.03.95**

(51) Int. Cl.[6] : **C07C 255/32,** C07C 255/47, C08K 5/16

(30) Priority : **18.03.94 US 210392**

(43) Date of publication of application : **20.09.95 Bulletin 95/38**

(84) Designated Contracting States : **BE DE FR GB IT NL**

(71) Applicant : **CIBA-GEIGY AG**
**Klybeckstrasse 141**
**CH-4002 Basel (CH)**

(72) Inventor : **Gande, Matthew E.**
**23 Ilion Road**
**New Fairfield, CT 06812 (US)**
Inventor : **Nesvadba, Peter**
**Route du Nord 5**
**CH-1723 Marly (CH)**
Inventor : **Pitteloud, Rita**
**Sur le Village**
**CH-1724 Praroman (CH)**

(54) **Stabilizers for the prevention of polymerization of (meth)acrylic acid and esters thereof.**

(57) The premature polymerization of acrylic acid, methacrylic acid and their esters and amides during isolation and processing is effectively suppressed or retarded by the presence of an effective amount of a selected diaryl substituted acetonitrile dimer stabilizer.

EP 0 672 652 A1

The premature polymerization of acrylic acid, methacrylic acid and their esters and amides during isolation and processing is effectively suppressed or retarded by the presence of an effective amount of a selected diaryl substituted acetonitrile dimer stabilizer.

## Background of the Invention

It is known that acrylic acid, methacrylic acid, and their esters and amides have a tendency to undergo unwanted polymerization when heated to elevated temperatures. The industrial production of such monomers also yields several byproducts, from which the desired monomer must be separated. The final stage of this purification is usually accomplished by distillation. During this final purification process, the monomer is heated and can undergo a thermally induced polymerization. This undesired reaction must be limited to insure that the reactors, tanks, and pipes used to make, store and transport the material remain free of high molecular weight polymeric material.

This problem has been addressed in a number of ways. Known inhibitors of such monomer polymerization include phenothiazine (PTZ), hydroquinone monomethyl ether, and methylene blue. U.S. Patent No. 5,221,764 disclose phenylenediamines with soluble metal salts and EP 522,709 describe aryl N-nitroso compounds as being active in inhibiting such monomer polymerization. However, there still remains a need for a more effective compound to improve the stability of such monomers during their distillation.

Phenothiazine, while unable to totally inhibit polymerization of such monomers, is a commonly used co-additive for polymerization inhibition. Any new stabilizer would accordingly preferably have to be compatible with it. Thus, potential stabilizers are commonly tested for any interaction with PTZ.

While air is present during the distillation process, there are often oxygen depleted zones within the distillation equipment. To ensure polymerization inhibition activity throughout the distillation train, a stabilizer must demonstrate activity under both aerobic and anaerobic conditions.

A variety of substituted diarylacetonitrile compounds have been synthesized and show considerable activity as polymer extrusion process stabilizers. A detailed description of these compounds is found in EP-A-0 608 198. While the "monomeric" diarylacetonitrile compounds are not radical polymerization inhibitors for the instant monomers, the "dimeric" compounds showed considerable activity in this end-use application. The activity demonstrated itself as either a retarding effect on the rate of polymerization, or as an long induction period before rapid polymerization occurs. The presence of oxygen does not enhance any such inhibition activity. Generally no interaction, either positive or negative, is observed between PTZ and the new class of inhibitors of formulae I, II and III.

## Detailed Disclosure

This invention pertains to the stabilization of acrylic acid and methacrylic acid and their esters and amides against premature polymerization during processing by the presence of an effective amount of a compound of formula I, II or III

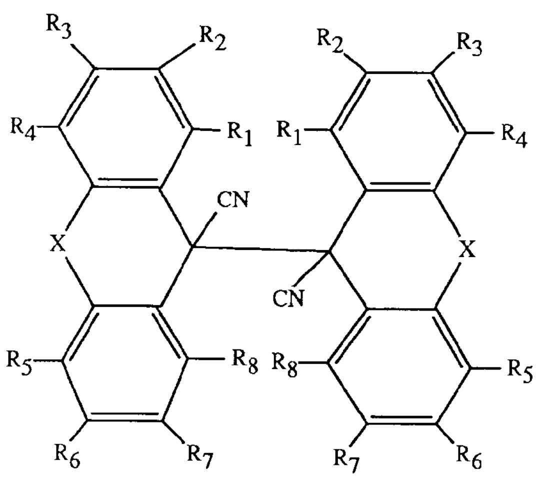

(I)

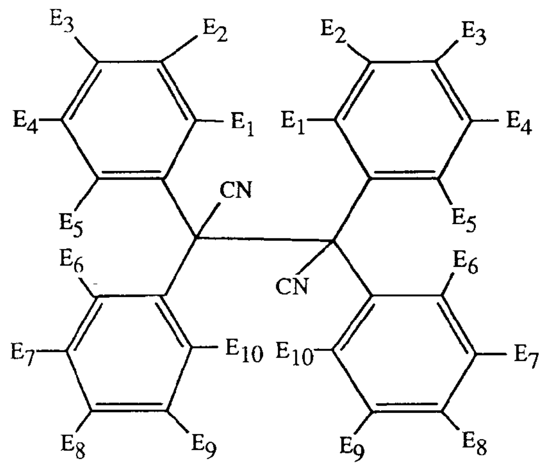

(II)

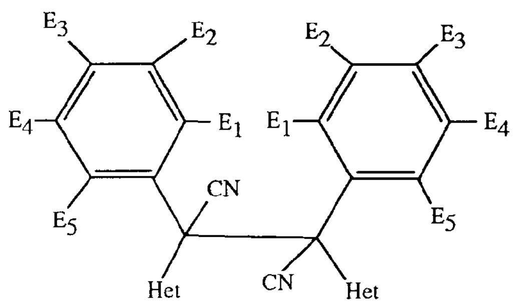

(III)

wherein

$R_1$, $R_2$, $R_3$, $R_4$, $R_5$, $R_6$, $R_7$ and $R_6$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $R_1$, $R_2$, $R_3$ and $R_4$, and at least one of $R_6$, $R_6$, $R_7$ and $R_8$ are hydrogen,

$E_1$, $E_2$, $E_3$, $E_4$, $E_5$, $E_6$, $E_7$, $E_8$, $E_9$ and $E_{10}$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $E_1$, $E_2$, $E_3$, $E_4$ and $E_5$, and at least one of $E_6$, $E_7$, $E_8$, $E_9$ and $E_{10}$ are hydrogen,

X is a direct bond, -O-, -S-, methylene, ethylene, vinylene or -$NR_9$- where $R_9$ is hydrogen, alkyl of 1 to 8 carbon atoms, benzyl, hydroxyl, alkoxy of 1 to 12 carbon atoms, alkanoyl of 2 to 12 carbon atoms or benzoyl, and

Het is heteroaryl.

Heteroaryl groups include 2-pyridyl, 3-pyridyl, 4-pyridyl, 2-thienyl, 3-thienyl, 4-quinolyl, 2-pyrimidyl, 4-quinaldinyl or pyrazinyl, especially 2-pyridyl or 4-pyridyl.

Preferably, $R_1$, $R_2$, $R_3$, $R_4$, $R_5$, $R_6$, $R_7$ and $R_6$, or $E_1$, $E_2$, $E_3$, $E_4$, $E_5$, $E_6$, $E_7$, $E_8$, $E_9$ and $E_{10}$ are independently hydrogen, alkyl of 1 to 12 carbon atoms, cycloalkyl of 5 to 6 carbon atoms, phenylalkyl of 7 to 9 carbon atoms, alkylthio of 1 to 8 carbon atoms or nitro.

Preferably, X is -O-, -S-, methylene, ethylene, vinylene or -$NR_9$- where $R_9$ is hydrogen or alkyl of 1 to 4 carbon atoms.

Most preferably, $R_1$, $R_2$, $R_3$, $R_4$, $R_5$, $R_6$, $R_7$ and $R_8$ or $E_1$, $E_2$, $E_3$, $E_4$, $E_5$, $E_6$, $E_7$, $E_8$, $E_9$ and $E_{10}$ are independently hydrogen, alkyl of 1 to 8 carbon atoms, phenylalkyl of 7 to 9 carbon atoms, alkylthio of 1 to 4 carbon atoms or nitro.

Most preferably, X is -O-, methylene or -$NR_9$- where $R_9$ is methyl.

Preferably, the acrylic compounds which can be stabilized by the instant compounds of formula **I**, **II** or **III** are acrylic compounds selected from the group consisting of acrylic acid, methacrylic acid, esters of acrylic acid or of methacrylic acid which are alkyl where alkyl is of 1 to 18 carbon atoms, cycloalkyl where cycloalkyl is cycloalkyl is of 5 to 12 carbon atoms, aliphatic bicyclic where the bicyclic group is of 6 to 10 carbon atoms,

acrylamide, methacrylamide, N,N-dimethylacrylamide and N,N-dimethylmethacrylamide.

More particularly, the acrylic compound is acrylic acid, methacrylic acid, an alkyl ester of acrylic acid or an alkyl ester of methacrylic acid.

Most preferably, the acrylic compound is acrylic acid.

The stabilizers of this invention are effective in preventing the premature polymerization of acrylic acid, methacrylic acid and their esters and amides at concentrations ranging from 0.0025 to 1.0% by weight, based on the weight of the monomer being stabilized. Preferably, the instant compounds are effective at the range of 0.005 to 0.5 % by weight.

The instant stabilizers are preferably selected from the group consisting of

9,9'-dicyano-9,9'-bis(xanthene);
9,9'-dicyano-9,9'-bis(thioxanthene);
9,9'-dicyano-9,9'-bis(9,10-dihydro-10-methylacridine);
2,2,3,3-tetraphenylsuccinonitrile; and
2,3-diphenyl-2,3-di(4-nitrophenyl)succinonitrile.

Most especially, the instant stabilizer is 2,2,3,3-tetraphenylsuccinonitrile or 2,3-diphenyl-2,3-di(4-nitrophenyl)succinonitrile.

The preferred embodiements described above apply to the stabilized composition as well as to the process for inhibiting premature polymerization.

The instant cyclic compounds are described in EP-A-0 608 198 along with a number of references describing their preparation such as U.S. Patent Nos. 2,956,063 and 3,452,076.

The "monomeric" 9-cyano structures can be dimerized by oxidation with iodine under alkaline conditions in an organic solvent at room temperature. Especially useful for this purpose is sodium methylate as the base and methanol, diethyl ether or tetrahydrofuran as the solvent.

The open chain diarylacetonitrile compounds are conveniently prepared from the an arene with the cyanohydrin of a benzaldehyde using a Friedel-Crafts catalyst to give the corresponding diarylacetonitrile. The diarylacetonitrile is then oxidized to the corresponding 2,2,3,3-tetraarylsuccinonitrile using iodine and a basic catalyst as discussed above.

An alternative method of making the diarylacetonitrile compound is the condensation of a benzyl cyanide with an activated haloaromatic compound.

Included in the scope of this invention is a new method of screening potential inhibitors and retarders of monomer radical polymerization which uses a mechanistic approach to the testing. This new method will allow for the determination of the effect of low levels of oxygen on the stabilization of the instant monomers to radical polymerization. Additives can be ranked based on time to a specific extent of monomer polymerization as well as observing their mode of action (i.e. inhibition with an induction period before observable polymerization, or retarding the rate of polymerization).

Several methods to determine the efficacy of potential stabilizers are used. One method, reported in United States Patent Nos. 5,171,882 and 5,221,461 involves simply heating a sample of monomeric material with a test stabilizer at a fixed temperature, usually between 80°C and 150°C, and determining the time to exotherm. A major drawback to this test is that under standard conditions, a well stabilized system may take up to 1 week to fail. Additionally, the reproducibility of the data obtained by this method is low (in one cited case, there was a ten-fold difference between two identical runs).

Laboratory scale distillations have also been used to test potential stabilizers (EP 0 522 709 A2). The drawback to this method is that it requires a much larger amount of monomer and requires a more detailed interpretation of the results (such as visual ranking of polymer build-up at various places in the system). This does not allow for a rapid screening of different classes of stabilizers.

Accordingly, a new screening test that involves the radical polymerization of monomer was developed to test a new class of potential polymerization inhibitors. The procedure involves the radical induced polymerization of a 3:1 mixture of acrylic acid and octyl acrylate in a low molecular weight carboxylic acid solvent. Free radicals were generated by the thermal decomposition of 2,2'-azobis(isobutyronitrile), AIBN, at 60°C. The degree of polymerization is determined by periodically measuring the solution viscosity, and comparing it to the initial viscosity. A four fold increase in viscosity is considered failure. Induction periods are determined by measuring the time before a significant change in viscosity is observed. To determine the retarding effect of a compound, the rate of increase of relative viscosity over time is compared to that of an unstabilized polymerization. The analysis time for this screening method is approximately three hours for a well stabilized sample.

The following examples are presented for the purpose of illustration only and are not to be construed to limit the scope or nature of the instant invention in any manner whatsoever.

Example 1

9,9'-Dicyano-9,9'-bis(xanthene)

To a solution of 20.7 g (0.1 mol) of 9-cyano-xanthene in 100 ml of tetrahydrofuran is added dropwise 19.4 ml (0.105 mol) of a 5.4 molar sodium methylate solution in methanol. The yellow solution is stirred for 10 minutes and than cooled to 5°C. To the cooled solution is added dropwise a solution of 13.0 g (0.051 mol) of iodine in 60 ml of tetrahydrofuran. The reaction mixture is then stirred for two hours at room temperature. The mixture is concentrated under vacuum, washed with water and extracted with ethyl acetate. The organic phase is washed with water and dried over anhydrous sodium sulfate. After concentration under vacuum, the crude product is recrystallized from toluene to give 7.5 g (36 % yield) of the title compound melting at 225°C.

The other instant compounds of formula I are made in analogous fashion from the corresponding "monomeric" 9-cyano compounds.

This method has been described generally by W. Hochstein et al, Liebigs Ann. Chem., 1823 (1978).

Example 2

Phenyl-(4-nitrophenyl)acetonitrile

The title compound is prepared by the reaction under basic conditions of benzyl cyanide and 1-chloro-4-nitrobenzene as taught by R. B. Davis et al., J. Org. Chem., 25, 1884 (1960).

Example 3

Phenyl-(2- or 4-pyridyl)acetonitrile

The general method of Example 2 can be used to prepare heterocyclic diarylacetonitriles by the similar reaction of benzyl cyanide with 2-chloropyridine or 4-chloropyridine to give the phenyl-(2-pyridyl)acetonitrile or phenyl-(4-pyridyl)acetonitrile as taught by Panizzon et al., Helv. Chim. Acta, 27, 1748 (1944).

Example 4

2,3-Diphenyl-2,3-di(4-pyridyl)succinonitrile

Following the general procedure of Example 1, the title compound is prepared by the dimerization of phenyl-(4-pyridyl)acetonitrile to give a compound melting at 137-139°C.

Examples 5-9

All compounds are tested under one of the following procedures:

Method A

A solution of monomers (3:1 weight ratio of acrylic acid to octyl acrylate) in acetic acid (0.1 g/mL) containing AIBN and additive to be tested (1% and 200 ppm, respectively, with respect to total monomer) is prepared. To a Canon-Fenske viscometer is added 10 mL of the solution, which is then purged with nitrogen for 10 minutes before being heated in a 60°C oil bath. Drop times are automatically measured (5 minute intervals between drops) using a Design Scientific automated viscometer and a custom software package.

Method B

A solution of monomers (3:1 weight ratio of acrylic acid to octyl acrylate) in propionic acid (0.1 g/mL) containing AIBN and additive to be tested (2% and 200 ppm, respectively, with respect to acrylate) is prepared. If compatibility with phenothiazine, PTZ, is being tested, PTZ is present at 125 ppm with respect to total monomers. To a Canon-Fenske viscometer is added 10 mL of the solution, which is then purged with nitrogen for 5 minutes before being heated in a 60°C oil bath. After an additional purge for 5 minutes, drop times are automatically measured (10 minute intervals, with a 1 minute nitrogen purge before each measurement) using a Design Scientific automated viscometer and a custom software package.

Method C

A solution of monomers (3:1 weight ratio of acrylic acid to octyl acrylate) in propionic acid (0.1 g/mL) containing AIBN and additive to be tested (2% and 400 ppm, respectively, with respect to acrylate) is prepared. To a Canon-Fenske viscometer is added 10 mL of the test solution, which is then purged with either nitrogen (>99.995%) or an oxygen gas mixture (6500 ppm oxygen in nitrogen) for 5 minutes before being heated in a 60°C oil bath. After an additional purge for 5 minutes, drop times are automatically measured (10 minute intervals, with a 1 minute gas purge before each measurement) using a Design Scientific automated viscometer and a custom software package.

The compounds are tested using one or more of the methods described above with the results being given in the Tables 1-5 below.

Table 1

Testing Results using Method A*

| Polymerization Inhibitor Compound** | Failure (minutes) | Induction Period (minutes) |
|---|---|---|
| None | 38 | - |
| A | 66 | 27 |
| B | 61 | 34 |
| C | 48 | 25 |
| D | 40 | 0 |
| E | 38 | 0 |

*The inhibitor compound is used at 200 ppm with respect to total monomer (0.1 g/mL) with 1 mg AIBN/mL of acetic acid at 60°C.

**A is 9,9'-dicyano-9,9'-bis(xanthene).
B is 2,2,3,3-tetraphenylsuccinonitrile.
C is 2,3-diphenyl-2,3-di(4-methylthiophenyl)succinonitrile.
D is 9-cyano-2-cumyl-4-methylxanthene.
E is 9-cyano-4-hexylxanthene.

It is clear from the results in Table 1 that the instant dimer compounds A, B and C are effective monomer polymerization inhibitors while the related "monomeric" compounds D and E are ineffective as polymerization inhibitors.

Table 2

Testing Results using Method B*

| Polymerization Inhibitor Compound** | Failure (minutes) | Induction Period (minutes) | Relative Polymerization Rate |
|---|---|---|---|
| None | 45 | - | 1.0 |
| A | 53 | 22 | 1.1 |
| F | 55 | 0 | 0.9 |
| G | 50 | 20 | 1.1 |
| H | 45 | 0 | 1.1 |

*The inhibitor compound is used at 200 ppm with respect to total monomer (0.1 g/mL) with 2 mg AIBN/mL of propionic acid at 60°C.

**A is 9,9'dicyano-9,9'-bis(xanthene).
F is 9,9'-dicyano-9,9'-bis(9,10-dihydro-10-methylacridine).
G is 2,3-diphenyl-2,3-di(4-nitrophenyl)succinonitrile.
H is 9-cyano-9,10-dihydro-10-methylacridine.

It is clear from the results in Table 2 that the instant dimer compounds A and G are effective monomer polymerization inhibitors while the related "monomeric" compound H is ineffective as a polymerization inhibitor. Compound F is a very effective retarder of polymerization as seen by the relative polymerization rate value.

The relative polymerization rate addresses whether a compound is a retarder as opposed to a true inhibitor of monomer polymerization. A low relative polymerization rate indicates retarding activity while a long induction period indicates an effective inhibitor.

Table 3

Testing Results using Method B*

(125 ppm of phenothiazine are present in each test)

| Polymerization Inhibitor Compound** | Failure (minutes) | Induction Period (minutes) | Relative Polymerization Rate |
|---|---|---|---|
| None | 49 | - | 1.0 |
| A | 59 | 21 | 1.0 |
| F | 82 | 0 | 0.6 |
| G | 59 | 23 | 1.0 |
| H | 51 | 0 | 1.1 |
| I | 50 | 0 | 1.0 |

*The inhibitor compound is used at 200 ppm with respect to total monomer (0.1 g/mL) with 2 mg AIBN/mL of propionic acid at 60°C.

**A is 9,9'dicyano-9,9'-bis(xanthene).
F is 9,9'-dicyano-9,9'-bis(9,10-dihydro-10-methylacridine).
G is 2,3-diphenyl-2,3-di(4-nitrophenyl)succinonitrile.
H is 9-cyano-9,10-dihydro-10-methylacridine.
I is 9,9'-dicyano-9,9'-bis(2-isopropylthioxanthene).

It is clear from the results in Table 3 that in the presence of phenothiazine the instant dimer compounds A and G are effective monomer polymerization inhibitors while the related "monomeric" compound H is ineffective as a polymerization inhibitor. Compound F is a very effective retarder of polymerization as seen by the relative polymerization rate value.

The relative polymerization rate addresses whether a compound is a retarder as opposed to a true inhibitor of monomer polymerization. A low relative polymerization rate indicates retarding activity while a long induction period indicates an effective inhibitor.

Table 4
Testing Results using Method C*

(these tests are run under nitrogen)

| Polymerization Inhibitor Compound** | Failure (minutes) | Induction Period (minutes) | Relative Polymerization Rate |
|---|---|---|---|
| None | 45 | - | 1.0 |
| A | 69 | 34 | 1.0 |
| F | 60 | 0 | 0.8 |
| I | 62 | 0 | 0.7 |

*The inhibitor compound is used at 400 ppm with respect to total monomer (0.1 g/mL) with 2 mg AIBN/mL of propionic acid at 60°C.

**A is 9,9'dicyano-9,9'-bis(xanthene)
F is 9,9'-dicyano-9,9'-bis(9,10-dihydro-10-methylacridine).
I is 9,9'-dicyano-9,9'-bis(2-isopropylthioxanthene).

It is clear from the results in Table 4 that under nitrogen the instant dimer compound A is a very effective monomer polymerization inhibitor. Instant compounds F and I are likewise very effective retarders of polymerization as seen by the relative polymerization rate values.

Table 5

Testing Results using Method C*

(these tests are run under 6500 ppm oxygen)

| Polymerization Inhibitor Compound** | Failure (minutes) | Induction Period (minutes) | Relative Polymerization Rate |
|---|---|---|---|
| None | 117 | - | 1.0 |
| A | 143 | - | 1.0 |
| F | 174 | 0 | 0.8 |

*The inhibitor compound is used at 400 ppm with respect to total monomer (0.1 g/mL) with 2 mg AIBN/mL of propionic acid at 60°C.

**A is 9,9'dicyano-9,9'-bis(xanthene)
F is 9,9'-dicyano-9,9'-bis(9,10-dihydro-10-methylacridine).

It is clear from the results in Table 5 that in the presence of oxygen the instant dimer compound A is a very effective monomer polymerization inhibitor. Instant compound F is a likewise very effective retarder of polymerization as seen by the relative polymerization rate value and failure time.

### Claims

1. A composition which comprises
   (a) an acrylic compound, and
   (b) an effective inhibiting amount, sufficient to prevent premature polymerization during distillation or purification of said acrylic compound, of a compound of formula I, II or III

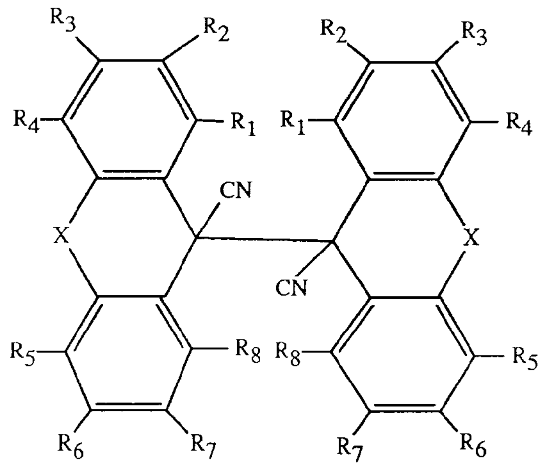

(I)

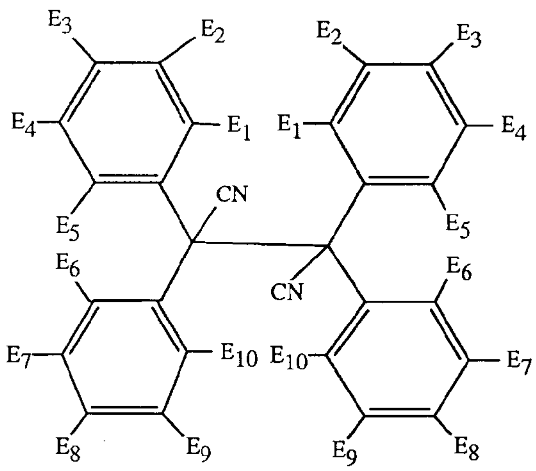

(II)

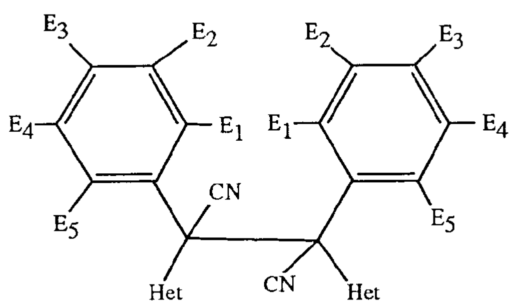

(III)

wherein

$R_1$, $R_2$, $R_3$, $R_4$, $R_5$, $R_6$, $R_7$ and $R_8$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $R_1$, $R_2$, $R_3$ and $R_4$, and at least one of $R_8$, $R_6$, $R_7$ and $R_8$ are hydrogen,

$E_1$, $E_2$, $E_3$, $E_4$, $E_5$, $E_6$, $E_7$, $E_8$, $E_9$ and $E_{10}$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $E_1$, $E_2$, $E_3$, $E_4$ and $E_5$, and at least one of $E_6$, $E_7$, $E_8$, $E_9$ and $E_{10}$ are hydrogen,

X is a direct bond, -O-, -S-, methylene, ethylene, vinylene or $-NR_9-$ where $R_9$ is hydrogen, alkyl of 1 to 8 carbon atoms, benzyl, hydroxyl, alkoxy of 1 to 12 carbon atoms, alkanoyl of 2 to 12 carbon atoms or benzoyl, and

Het is heteroaryl.

**2.** A composition according to claim 1 wherein the effective amount of component (b) is 0.0025 to 1.0 % by weight, based on the weight of the acrylic compound of component (a).

**3.** A composition according to claim 2 wherein the effective amount of component (b) is 0.005 to 0.5 % by weight.

**4.** A composition according to claim 1 wherein the acrylic compound of component (a) is selected from the group consisting of acrylic acid, methacrylic acid, esters of acrylic acid or of methacrylic acid which are alkyl where alkyl is of 1 to 18 carbon atoms, cycloalkyl where cycloalkyl is cycloalkyl is of 5 to 12 carbon atoms, aliphatic bicyclic where the bicyclic group is of 6 to 10 carbon atoms, acrylamide, methacrylamide, N,N-dimethylacrylamide and N,N-dimethylmethacrylamide.

**5.** A composition according to claim 1 wherein $R_1$, $R_2$, $R_3$, $R_4$, $R_5$, $R_6$, $R_7$ and $R_6$, or $E_1$, $E_2$, $E_3$, $E_4$, $E_5$, $E_6$, $E_7$, $E_8$, $E_9$ and $E_{10}$ are independently hydrogen, alkyl of 1 to 12 carbon atoms, cycloalkyl of 5 to 6 carbon atoms, phenylalkyl of 7 to 9 carbon atoms, alkylthio of 1 to 8 carbon atoms or nitro.

**6.** A composition according to claim 1 where in the compound of formula I, X is -O-, -S-, methylene, ethylene, vinylene or $-NR_9-$ where $R_9$ is hydrogen or alkyl of 1 to 4 carbon atoms.

**7.** A composition according to claim 1 wherein X is -O-, methylene or $-NR_9-$ where $R_9$ is methyl.

**8.** A composition according to claim 1 wherein the compound of component (b) is selected from the group consisting of

9,9'-dicyano-9,9'-bis(xanthene);
9,9'-dicyano-9,9'-bis(thioxanthene);
9,9'-dicyano-9,9'-bis(9,10-dihydro- 10-methylacridine);
2,2,3,3-tetraphenylsuccinonitrile; and
2,3-diphenyl-2,3-di(4-nitrophenyl)succinonitrile.

**9.** A process for inhibiting the premature polymerization of an acrylic compound (component (a)) during distillation or purification which comprises

incorporating therein an effective inhibiting amount, sufficient to prevent premature polymerization during distillation or purification of said acrylic compound, of a compound of formula I, II or III (component (b))

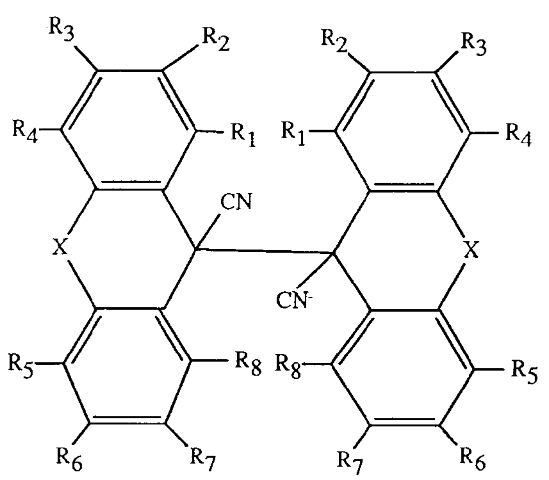

(I)

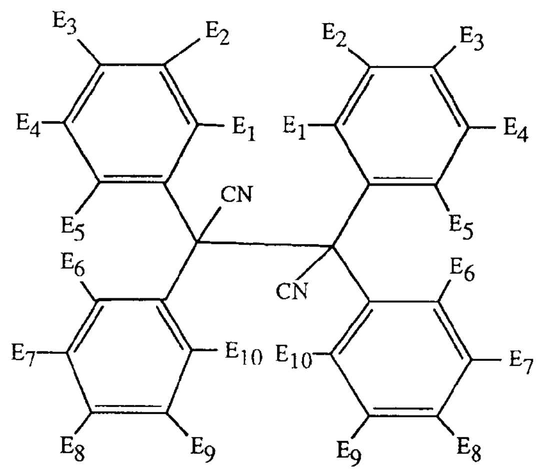

(II)

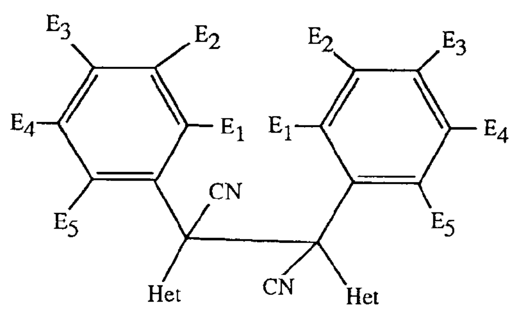

(III)

wherein

$R_1$, $R_2$, $R_3$, $R_4$, $R_5$, $R_6$, $R_7$ and $R_8$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $R_1$, $R_2$, $R_3$ and $R_4$, and at least one of $R_8$, $R_6$, $R_7$ and $R_8$ are hydrogen,

$E_1$, $E_2$, $E_3$, $E_4$, $E_5$, $E_6$, $E_7$, $E_8$, $E_9$ and $E_{10}$ are independently hydrogen, chloro, alkyl of 1 to 18 carbon atoms, cycloalkyl of 5 to 8 carbon atoms, phenyl, phenylalkyl of 7 to 9 carbon atoms, hydroxy, alkoxy of 1 to 18 carbon atoms, alkylthio of 1 to 18 carbon atoms, alkanoyloxy of 1 to 18 carbon atoms, alkenoyloxy of 3 to 18 carbon atoms, benzoyloxy, cyano, nitro, amino, alkylamino of 1 to 8 carbon atoms or dialkylamino of 1 to 8 carbon atoms, where at least one of $E_1$, $E_2$, $E_3$, $E_4$ and $E_5$, and at least one of $E_6$, $E_7$, $E_8$, $E_9$ and $E_{10}$ are hydrogen,

X is a direct bond, -O-, -S-, methylene, ethylene, vinylene or $-NR_9-$ where $R_9$ is hydrogen, alkyl of 1 to 8 carbon atoms, benzyl, hydroxyl, alkoxy of 1 to 12 carbon atoms, alkanoyl of 2 to 12 carbon atoms or benzoyl, and

Het is heteroaryl.

**10.** A process according to claim 9 wherein the effective amount of component (b) is 0.0025 to 1.0 % by weight, based on the acrylic monomer of component (a).

**11.** A process according to claim 9 wherein the effective amount of component (b) is 0.005 to 0.5 % by weight, based on the acrylic monomer of component (a).

**12.** A method for assessing the effectiveness of a compound to inhibit or retard the premature polymerization of an acrylic monomer which comprises

dissolving 200 to 400 ppm of said compound, 1-2% by weight of 2,2'-azobis(isobutyronitrile) and 10% by weight of the acrylic test monomer or mixture of acrylic test monomers in a lower alkanoic acid solvent,

adding the above solution to a Canon-Fenske viscometer,

purging the viscometer with an appropriate gas for 5 to 10 minutes,
heating the viscometer in a 60°C oil bath, and
measuring the drop times of the solution from the viscometer at 5 to 10-minute intervals, and
ascertaining the induction periods and calculating the relative polymerization rates from said drop time information.

European Patent Office

# EUROPEAN SEARCH REPORT

Application Number
EP 95 81 0154

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | MAKROMOL. CHEM., vol. 184, 1983 pages 287-294, A. BLEDZKI ET AL. 'Polymerisationsauslösung mit substituierten Ethanen, 5: Polymerisation von verschiedenen Methacrylmonomeren.' * page 291 * * figure 2 * --- | 1,4,5 | C07C255/32<br>C07C255/47<br>C08K5/16 |
| D,A | EP-A-0 522 709 (UNIROYAL CHEMICAL COMPANY) * the whole document * --- | 1 | |
| D,A | US-A-5 221 764 (ROLING) * the whole document * --- | 1 | |
| D,P,A | EP-A-0 608 198 (CIBA.GEIGY AG) * page 2, line 12 - page 3, line 23 * * page 9, line 57 - line 58 * * page 11, line 5 - line 7 * *compound 118* * page 21, line 1 - line 23 * --- | 1 | TECHNICAL FIELDS SEARCHED (Int.Cl.6)<br>C07C<br>C08K |
| X | GB-A-1 167 666 (DU PONT DE NEMOURS) * example 1 * ----- | 12 | |

The present search report has been drawn up for all claims

| Place of search | Date of completion of the search | Examiner |
|---|---|---|
| MUNICH | 16 June 1995 | Goetz, G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)